# EUROPEAN PATENT SPECIFICATION

(11) **EP 0 404 454 B1**
(45) Date of publication and mention of the grant of the patent: **18.08.1993**
(21) Application number: 90306537.3
(22) Date of filing: 15.06.1990
(51) Int. Cl.: A61M 15/00

(54) **Disposable medicament inhalation device**
Wegwerfvorrichtung zur Inhalation von Arzneimitteln
Dispositif à utilisation unique pour l'inhalation de médicaments

(30) Priority: 21.06.1989 GB 8914223
(43) Date of publication of application: 27.12.1990
(73) Proprietor: FISONS plc, Ipswich Suffolk IP1 1QH (GB)
(72) Inventor: Chawla, Brindra Paul Singh, West Bridgford, Nottingham NG2 7BZ (GB); Clark, Andrew Reginald, Loughborough, Leicestershire (GB)
(74) Representative: Craig, Christopher Bradberry

(56) References cited:
- WO-A-82/01470
- CH-A- 662 277
- DE-A- 2 118 842
- GB-A- 2 165 159
- GB-A- 2 179 260
- US-A- 2 533 065
- US-A- 4 265 236

## Description

This invention relates to a device for the administration of powdered inhalation medicaments, more particularly to such a device which is used for a small number of administrations (for example one) and is then disposed of.

Reusable devices for the administration of powdered inhalation medicaments are well known. For example, GB-A-1122284 discloses a device for the administration of medicament held in a gelatin capsule, which comprises a capsule-puncturing mechanism and a propeller-shaped capsule holder which rotates during inhalation by a patient to empty medicament from the punctured capsule. Although the device has proved most beneficial to some patients, it is too complicated for others to operate effectively. In addition, the device is expensive to manufacture, and requires regular cleaning of its components which entails disassembly and reassembly of the device.

In order to overcome the disadvantages of reusable devices, a number of disposable inhalation devices have been proposed. GB-A-2179260 discloses such a device comprising a body defining an endless path communicating with an air inlet and an air outlet. A ball is provided within the endless path which travels around the path upon inhalation by a patient, thus releasing medicament coated on the ball or on the inner surface of the endless path into the inhaled airstream. The device has the disadvantages that the endless path is difficult to manufacture which consequently makes the device expensive, and that some medicaments are not delivered satisfactorily from it, notably those which have a unit dose of comparatively large mass. In addition, there is a danger that the dose delivered to a patient from such a device may vary widely since it will depend upon attrition of the coating by impaction of the ball on the surface. The dose delivered may therefore depend, amongst other things, upon the force with which air is inhaled through the device.

US-A-4265236 discloses a disposable nasal inhalation device comprising a length of flexible tubing in which medicament is held, which is formed into a closed loop prior to use. In use, the ends of the tube are separated and a patient inhales through the tube, thus entraining the medicament into the inhaled air. In order to produce the air turbulence necessary to break up aggregations of powder, a preferred form of the device is provided with a small length of pipe within the tube, the pipe having spiral ridges on its inner surface. Not only does this feature increase the cost of the device, but there is also a danger that the pipe may become dislodged and be inhaled by a patient with potentially disastrous consequences. In addition, because of the open nature of the device, there is a risk that powder may fall out of the device between separation of the two ends of the tube and inhalation.

US-A-2533065 discloses inter alia a disposable inhalation device wherein the dose of powdered medicament is held in a cylindrical cup to which is attached a cap through which the patient inhales. The presence of the separate medicament reservoir and cap detracts significantly from the compactness of the device.

We have now devised a disposable inhalation device which overcomes or substantially mitigates the disadvantages of prior art devices.

According to the present invention, there is provided a disposable device for the administration of powdered inhalation medicament comprising a base member; a rigid medicament reservoir holding a unit dose of medicament in loose powder form and having air inlet means comprising an air passageway which connects one or more openings in the surface of the device with the medicament reservoir; medicament outlet means; and closure means for the inlet and outlet means which are removable in use; characterized in that the medicament reservoir and the medicament outlet means are situated in a portion of the device raised from the base member which is adapted to fit between a user's lips.

Devices according to the invention have the advantages that they are sufficiently cheap to be disposable, do not have components which could be dislodged and inhaled during use, sufficiently enclose the medicament to prevent it falling out of the device immediately before use, are very simple to use, and are capable of delivering an accurate dose of medicament to a patient.

The air inlet means may comprise one or more openings having a total inlet cross sectional area, and the medicament outlet means may similarly comprise one or more openings having a total outlet cross sectional area. The ratio of the total outlet cross sectional area to the total inlet cross sectional area is preferably in the range of from 1:1 to 1:10, more preferably from 1:2 to 1:4, for example 1:3. We have found that cross sectional areas in the preferred ratio aid break-up of powder aggregates.

The raised portion of the device adapted to fit between a user's lips may be, for example, hemispherical or distorted hemispherical having an oval lateral cross section, so that the raised portion may act as a mouthpiece.

We prefer the medicament outlet means to comprise a group of openings in a wall of the reservoir, for example the openings may be arranged in a grid. Grids are preferred because they break up any aggregates of powder as they pass through, and they also retain powder in the device immediately before administration more effectively than a single opening of comparable cross sectional area.

The air passageway which connects one or more openings in a surface of the device with the medicament reservoir may join the reservoir tangentially. Two such passageways could be provided on opposite sides of the reservoir so that a swirling motion is imparted to the air in the reservoir during inhalation through the device by a patient.

It is possible to produce the device with very small dimensions. This has the advantages that the medicament may be administered discreetly without embarrassment, very little material is required to produce it thus reducing costs and waste, and a day's dose may be carried conveniently in a small volume. However, when the device is to be used by patients who might swallow it, the body may be of enlarged dimensions, and such a body could be provided with a sufficient number of reservoirs to provide one day's supply of medicament to a patient, for example four. Of course, such a device might be preferred by any patient for yet greater compactness.

Devices according to the invention are useful for the administration of inhalation medicaments where there is a comparatively large mass of active ingredient in each dose, for example sodium cromoglycate and nedocromil sodium, where unit doses are often several milligrams in mass and other disposable devices are not suitable to administer them. However, devices according to the present invention may also be used to administer inhalation medicaments where a comparatively small mass of active ingredient is delivered in each dose, for example steroidal drugs where each unit dose is several hundred micrograms in mass.

The inner surface of the reservoir may be shaped so as to produce air turbulence in use, thus aiding break-up of powder aggregates and dispersion of the powder. For example, a baffle may be provided in the reservoir adjacent to the point at which the air inlet means join it.

Devices according to the invention may be manufactured from plastics material using conventional forming methods, for example vacuum forming or injection moulding.

We prefer the closure means to be a cover sheet which is removably attached to an outer surface of the device. Such a cover sheet preferably seals both the openings of the air inlet means and the openings of the medicament outlet means. Advantageously, the openings of the air inlet means and the medicament outlet means are situated on one face of the device, in which case the cover sheet need only be of comparably small size.

The closure means may be made of plastics material, metal foil, or a laminate of plastics and metal foil. It may be held on the container by any convenient means, for example glue or heat sealing, and is preferably removable by hand in one piece.

As well as retaining the medicament, the closure means may seal the container from contamination by microorganisms. Thus a sterile device is assured for each administration.

A preferred embodiment of the present invention will now be described, by way of example, with reference to the accompanying drawings, in which:

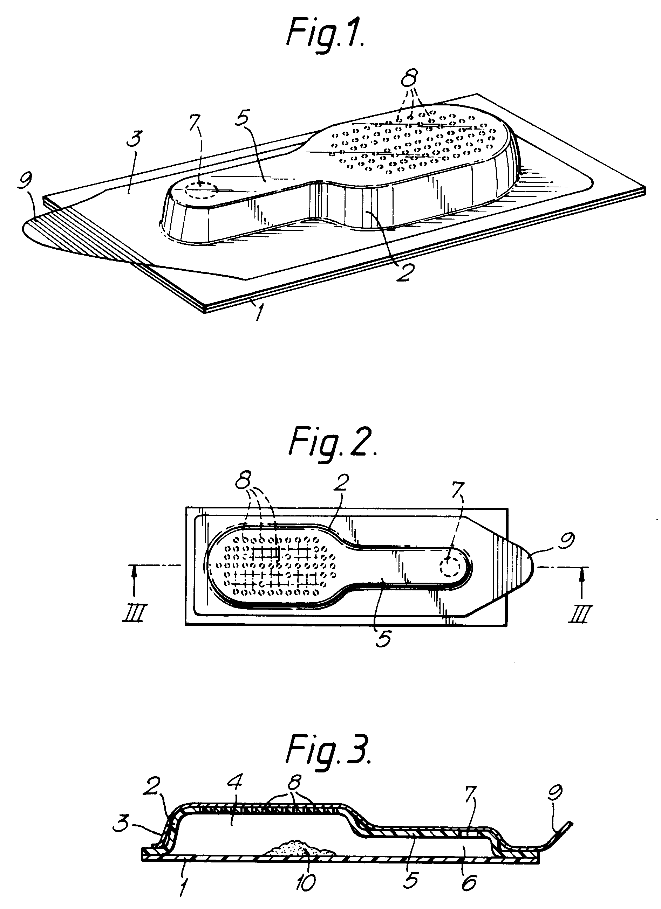

Figure 1 is a perspective view of a disposable inhalation device according to the invention;
Figure 2 is a plan view of the device of Figure 1; and
Figure 3 is a cross sectional view along the line III-III of Figure 2.

A disposable inhalation device according to the invention comprises a flat plastics rectangular base 1 heat-sealed to a formed plastics upper portion 2, and a removable plastics-metal laminate cover sheet 3 glued to the outer surface of upper portion 2.

Upper portion 2 defines a medicament reservoir 4 having an arm 5 extending from one end. Arm 5 defines an air passageway 6 connecting an air inlet 7 at one end of the arm with medicament reservoir 4 at the other end.

A number of holes 8 arranged in a grid and opening into medicament reservoir 4 are provided on the surface of upper portion 2. This upper surface is shaped so as to fit comfortably between a patient's lips so that it may act as a mouthpiece.

When cover sheet 3 is attached to the device, air inlet 7 and holes 8 are sealed prior to use.

To use the device, a patient simply pulls on a tab portion 9 of cover sheet 3, which consequently peels away from the surface of upper portion 2 to open air inlet 7 and holes 8. The mouthpiece is then offered up to the patient's lips, whereupon inhalation through holes 8 results in air being drawn into air inlet 7, along passageway 6 and into medicament reservoir 4 where unit dose of medicament 10 is entrained by the airstream. The entrained medicament then passes out of the device through holes 8 and is administered to the patient.

## Claims

1. A disposable device for the administration of powdered inhalation medicament comprising a base member (1); a rigid medicament reservoir (4) holding a unit dose of medicament (10) in loose powder form and having air inlet means comprising an air passageway (6) which connects one or more openings (7) in the surface of the device with the medicament reservoir (4); medicament outlet means (8); and closure means (3) for the inlet and outlet means which are removable in use; characterized in that the medicament reservoir (4) and the medicament outlet means (8) are situated in a portion of the device raised from the base member (1) which is adapted to fit between a user's lips.

2. A disposable device according to claim 1, wherein the air inlet means comprise one or more openings (7) having a total inlet cross sectional area and the medicament outlet means (8) comprise one or more openings having a total outlet cross sectional area; further characterized in that the ratio of the total outlet cross sectional area to the total inlet cross sectional area is in the range of from 1:1 to 1:10.

3. A disposable device according to any one of the preceding claims, further characterized in that the medicament outlet means (8) comprise a group of openings in a wall of the reservoir (4).

4. A disposable device according to any one of the preceding claims, further characterized in that the device is provided with a sufficient number of reservoirs (4) to provide one day's supply of medicament to a patient.

5. A disposable device according to any one of the preceding claims, further characterized in that the medicament (10) held by the reservoir (4) is selected from sodium cromoglycate and nedocromil sodium.

6. A disposable device according to any one of the preceding claims, further characterized in that an inner surface of the reservoir (4) is shaped so as to produce air turbulence in use.

7. A disposable device according to any one of the preceding claims, further characterized in that the closure means (3) is a cover sheet removably attached to an outer surface of the device.

## Patentansprüche

1. Einwegvorrichtung zur Verabreichung eines pulverförmigen Inhalationsmedikaments, welche einen Basisteil (1); einen unbiegsamen Medikamentenbehälter (4), der eine Einheitsdosis eines Medikaments (10) in Form eines losen Pulvers faßt und Lufteinlaßmittel hat, die einen Luftströmungsweg (6) aufweisen, der eine oder mehrere Öffnungen (7) in der Oberfläche der Vorrichtung mit dem Medikamentenbehälter (4) verbindet; Medikament-Auslaßmittel (8); und ein beim Gebrauch abnehmbares Verschlußmittel (3) für die Einlaß- und Auslaßmittel umfaßt; dadurch gekennzeichnet, daß der Medikamentenbehälter (4) und die Medikament-Auslaßmittel (8) in einem Teil der Vorrichtung angeordnet sind, der vom Basisteil (1) erhöht ist und der geeignet ist, um zwischen die Lippen eines Benutzers zu passen.

2. Einwegvorrichtung nach Anspruch 1, bei welcher die Lufteinlaßmittel eine oder mehrere Öffnungen (7) mit einer Einlaß-Gesamtquerschnittsfläche aufweisen, und die Medikament-Auslaßmittel (8) eine oder mehrere Öffnungen mit einer Auslaß-Gesamtquerschnittsfläche aufweisen; ferner dadurch gekennzeichnet, daß das Verhältnis der Auslaß-Gesamtquerschnittsfläche zur Einlaß-Gesamtquerschnittsfläche im Bereich von 1:1 bis 1:10 liegt.

3. Einwegvorrichtung nach einem der vorhergehenden Ansprüche, ferner dadurch gekennzeichnet, daß die Medikament-Auslaßmittel (8) eine Gruppe von Öffnungen in einer Wand des Behälters (4) aufweisen.

4. Einwegvorrichtung nach einem der vorhergehenden Ansprüche, ferner dadurch gekennzeichnet, daß die Vorrichtung mit einer ausreichenden Anzahl von Behältern (4) versehen ist, um einen Tages-Medikamentenbedarf für einen Patienten vorzusehen.

5. Einwegvorrichtung nach einem der vorhergehenden Ansprüche, ferner dadurch gekennzeichnet, daß das vom Behälter (4) gehaltene Medikament (10) aus Natriumcromoglykat und Natriumnedocromil ausgewählt ist.

6. Einwegvorrichtung nach einem der vorhergehenden Ansprüche, ferner dadurch gekennzeichnet, daß eine Innenfläche des Behälters (4) derart geformt ist, um beim Gebrauch Luftturbulenzen zu erzeugen.

7. Einwegvorrichtung nach einem der vorhergehenden Ansprüche, ferner dadurch gekennzeichnet, daß das Verschlußmittel (3) eine Abdeckfolie ist, die an einer Außenfläche der Vorrichtung ablösbar angebracht ist.

## Revendications

1. Dispositif jetable pour l'administration par inhalation d'un médicament en poudre comprenant un élément de base (1), un réservoir de médicament rigide (4) contenant une dose unitaire de médicament (10) sous forme d'une poudre meuble et comportant des moyens d'entrée d'air comprenant un passage d'air (6) qui raccorde une ou plusieurs ouvertures (7) dans la surface du dispositif au réservoir de médicament (4), des moyens de sortie de médicament (8), et des moyens d'obturation (3) pour les moyens d'entrée et de sortie qui sont amovibles en service, caractérisé en ce que le réservoir de médicament (4) et les moyens de sortie de médicament (8) sont situés dans une partie du dispositif surélevée par rapport à l'élément de base (1) et conçue pour s'ajuster entre les lèvres de l'utilisateur.

2. Dispositif jetable suivant la revendication 1, dans lequel les moyens d'entrée d'air comprennent une ou plusieurs ouvertures (7) présentant une aire de section transversale d'entrée totale et les moyens de sortie de médicament (8) comprennent une ou plusieurs ouvertures présentant une aire de section transversale de sortie totale, caractérisé en outre en ce que le rapport de l'aire de section transversale de sortie totale a l'aire de section transversale d'entrée totale se situe dans l'intervalle allant de 1:1 à 1:10.

3. Dispositif jetable suivant l'une quelconque des revendications précédentes, caractérisé en outre en ce que les moyens de sortie de médicament (8) comprennent un groupe d'ouvertures dans une paroi du réservoir (4).

4. Dispositif jetable suivant l'une quelconque des revendications précédentes, caractérisé en outre en ce qu'il est pourvu d'un nombre suffisant de réservoirs (4) pour fournir une réserve journalière de médicament à un patient.

5. Dispositif jetable suivant l'une quelconque des revendications précédentes, caractérisé en outre en ce que le médicament (10) retenu par le réservoir (4) est du cromoglycate de sodium ou du nédocromil sodique.

6. Dispositif jetable suivant l'une quelconque des revendications précédentes, caractérisé en outre en ce qu'une surface intérieure (4) est façonnée de manière à produire une turbulence d'air en service.

7. Dispositif jetable suivant l'une quelconque des revendications précédentes, caractérisé en outre en ce que les moyens d'obturation (3) comprennent une feuille de recouvrement attachée de manière amovible à une surface extérieure du dispositif.
